# EUROPEAN PATENT APPLICATION

(11) **EP 4 095 137 A1**
(43) Date of publication of application: **30.11.2022**
(21) Application number: 21753616.8
(22) Date of filing: 10.02.2021
(51) Int. Cl.: C07D 471/04, A61K 31/519, A61P 35/00

(54) **USE OF PYRIDO[1,2-A]PYRIMIDINONE COMPOUND IN TREATING LYMPHOMA**

(30) Priority: 10.02.2020 CN 202010084226; 10.02.2020 CN 202010084209; 10.02.2020 CN 202010084222; 15.09.2020 CN 202010967167
(71) Applicant: Chia Tai Tianqing Pharmaceutical Group Co., Ltd., Lianyungang City, Jiangsu 222062 (CN)
(72) Inventor: FENG, Fan, Nanjing City, Jiangsu 211100 (CN); WANG, Xunqiang, Nanjing City, Jiangsu 211100 (CN); CHEN, Li, Nanjing City, Jiangsu 211100 (CN); HAN, Xi, Nanjing City, Jiangsu 211100 (CN); WU, Naiying, Nanjing City, Jiangsu 211100 (CN); MA, Ruiting, Nanjing City, Jiangsu 211100 (CN); YANG, Chaoqiang, Nanjing City, Jiangsu 211100 (CN)
(74) Representative: Thoma, Michael
(86) International application number: PCT/CN2021/076423
(87) International publication number: WO 2021/160147

(57) **Abstract**

The present application belongs to the field of medicinal chemistry, and relates to a use of a pyrido[1,2-a]pyrimidinone compound in treating lymphoma. Specifically, the present application relates to a pyrido[1,2-a]pyrimidinone compound or a pharmaceutical composition thereof for treating lymphoma, and a method or use of pyrido[1,2-a]pyrimidinone compound in treating lymphoma.

## Description

### CROSS-REFERENCE TO RELATED APPLICATIONS

The present application claims the priority and benefit to the Chinese Patent Application No. 202010084226.X filed with China National Intellectual Property Administration on February 10, 2020, the Chinese Patent Application No. 202010084209.6 filed with China National Intellectual Property Administration on February 10, 2020, the Chinese Patent Application No. 202010084222.1 filed with China National Intellectual Property Administration on February 10, 2020, and the Chinese Patent Application No. 202010967167.0 filed with China National Intellectual Property Administration on September 15, 2020, the content of each of which is incorporated herein by reference in its entirety.

### TECHNICAL FIELD

The present application belongs to the field of medicinal chemistry, and relates to use of a pyrido[1,2-a]pyrimidinone compound in treating lymphoma.

### BACKGROUND

PI3K pathway is the most frequently mutated part in cancer cells of the human body, which can lead to proliferation, activation and signal amplification of cells.

PI3K kinase (phosphatidylinositol-3-kinase, PI3Ks) belongs to the lipid kinase family and can phosphorylate 3'-OH end of the inositol ring of phosphatidylinositol. The PI3K kinase is a lipid kinase consisting of a regulatory subunit p85 or p101 and a catalytic subunit p110, and activates downstream Akt and the like by catalyzing phosphorylation of phosphatidylinositol 4,5-bisphosphate (PIP2) to phosphatidylinositol 3,4,5-trisphosphate (PIP3), thereby playing a key role in proliferation, survival, metabolism and the like of cells. Therefore, inhibiting the phosphatidylinositol-3-kinase may affect the PI3K pathway, thereby inhibiting proliferation and activation of cancer cells.

The tumor suppressor gene PTEN (phosphatase and tension homolog deleted on chromosome ten) enables PIP3 to be dephosphorylated to generate PIP2, thereby achieving negative regulation of PI3K/Akt signaling pathway, inhibiting proliferation of cells and promoting apoptosis of the cells. The frequent occurrences of PI3K gene mutation and amplification in cancers, absence of PTEN in cancers and the like all suggest that PI3K is closely related to tumorigenesis.

A series of compounds as PI3K inhibitors are disclosed in WO2015192760, and a compound of formula I with the following structure is also specifically disclosed:

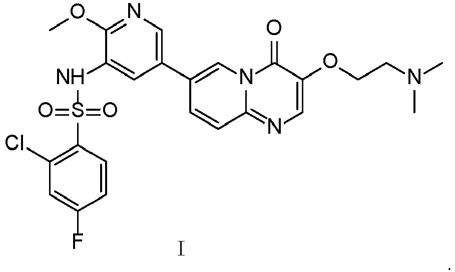

### SUMMARY

The present application provides a compound of formula I or a pharmaceutically acceptable salt thereof for use in treating lymphoma in a patient:

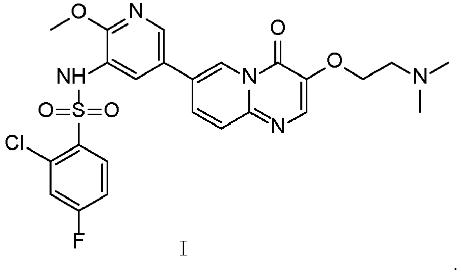

In another aspect, the present application provides use of the compound of formula I or the pharmaceutically acceptable salt thereof in preparing a medicament for treating lymphoma in a patient.

In another aspect, the present application provides use of the compound of formula I or the pharmaceutically acceptable salt thereof in treating lymphoma in a patient.

In another aspect, the present application provides a method for treating lymphoma in a patient, which comprises administering to the patient the compound of formula I or the pharmaceutically acceptable salt thereof.

In some embodiments of the present application, the compound of formula I or the pharmaceutically acceptable salt thereof disclosed herein is used as a single active agent.

In some embodiments of the present application, the compound of formula I or the pharmaceutically acceptable salt thereof disclosed herein can be in a form of a pharmaceutical composition comprising a therapeutically effective amount of the compound of formula I or the pharmaceutically acceptable salt thereof.

In another aspect, the present application provides a pharmaceutical composition comprising the compound of formula I or the pharmaceutically acceptable salt thereof as an active ingredient for use in treating lymphoma.

### DETAILED DESCRIPTION OF INVENTION

### Lymphoma

In some embodiments of the present application, the lymphoma is selected from the group consisting of Hodgkin's lymphoma (HL) and non-Hodgkin's lymphoma (NHL).

In some embodiments of the present application, the lymphoma is selected from the group consisting of B-cell lymphoma and T-cell lymphoma. In some embodiments of the present application, the lymphoma is selected from the group consisting of classical Hodgkin's lymphoma (CHL), nodular lymphocyte Hodgkin's lymphoma, mantle cell lymphoma (MCL), follicular lymphoma (FL), diffuse large B-cell lymphoma (DLBCL), small lymphocytic lymphoma (SLL) and chronic lymphocytic leukemia (CLL).

In some embodiments of the present application, the non-Hodgkin's lymphoma is selected from the group consisting of B-cell lymphoma and T-cell lymphoma. In some embodiments of the present application, the non-Hodgkin's lymphoma is selected from the group consisting of mantle cell lymphoma (MCL), follicular lymphoma (FL), diffuse large B-cell lymphoma (DLBCL), small lymphocytic lymphoma (SLL) and chronic lymphocytic leukemia (CLL).

In some embodiments of the present application, the mantle cell lymphoma is selected from the group consisting of classical mantle cell lymphoma, leukemic non-nodal mantle cell lymphoma and *in situ* mantle cell neoplasia.

In some embodiments of the present application, the diffuse large B-cell lymphoma is selected from the group consisting of germinal center B-cell-like (GCB) diffuse large B-cell lymphoma, activated B-cell-like (ABC) diffuse large B-cell lymphoma and Type 3 diffuse large B-cell lymphoma.

In some embodiments of the present application, the Hodgkin's lymphoma is selected from the group consisting of classical Hodgkin's lymphoma and nodular lymphocyte Hodgkin's lymphoma. In some embodiments of the present application, the classical Hodgkin's lymphoma is selected from the group consisting of nodular sclerosis classical Hodgkin's lymphoma, lymphocyte-rich classical Hodgkin's lymphoma, mixed cellularity classical Hodgkin's lymphoma and lymphocyte-depleted classical Hodgkin's lymphoma.

In some embodiments of the present application, the patient with lymphoma has received treatment with one or more prior therapeutic regimens. In some embodiments of the present application, the patient with the lymphoma has received treatment with one, two, three, four or five prior therapeutic regimens.

In some embodiments of the present application, the patient with the lymphoma is one who has received treatment with first-line, second-line, or ≥ third-line prior therapeutic regimen.

In some embodiments of the present application, the lymphoma is selected from relapsed or refractory lymphoma. In some embodiments of the present application, the disease reoccurs after the patient with the lymphoma has received treatment with the prior therapeutic regimen and achieved objective response, or the patient with the lymphoma has no objective response for the prior therapeutic regimen. In some embodiments of the present application, the no objective response refers to stable disease or disease progression during treatment.

In some embodiments of the present application, the patient with the lymphoma is a patient with relapsed or refractory lymphoma who has received treatment with the prior therapeutic regimen.

In some embodiments of the present application, the patient with the lymphoma is a patient with CD20-positive (CD20+), CD30-positive (CD30+), CD38-positive (CD38+) and/or ZAP70-positive (ZAP70+) lymphoma.

In some embodiments of the present application, the patient with the lymphoma is a patient with CD20-positive (CD20+) lymphoma.

In some embodiments of the present application, the patient with the lymphoma is one who has received treatment with rituximab and/or a BTK inhibitor.

In some embodiments of the present application, the patient with the lymphoma is a patient with relapsed or refractory lymphoma who has received treatment with rituximab and/or a BTK inhibitor.

In some embodiments of the present application, the patient with the lymphoma is CD20-positive (CD20+) and is a patient with relapsed or refractory lymphoma who has received treatment with rituximab.

In some embodiments of the present application, the prior therapeutic regimens comprise drug therapy, radiotherapy or hematopoietic stem cell transplantation.

In some embodiments of the present application, the drug therapy comprises interferon therapy, chemotherapy or targeted drug therapy.

In some embodiments of the present application, the targeted drug therapy comprises an anti-CD20 antibody, an anti-CD30 antibody or a BTK inhibitor.

In some embodiments of the present application, the anti-CD20 antibody comprises rituximab, CHO-H01, ocaratuzumab, ibritumomab tiuxetan, ublituximab or obinutuzumab.

In some embodiments of the present application, the anti-CD30 antibody can comprise brentuximab vedotin.

In some embodiments of the present application, the BTK inhibitor comprises ibrutinib, ICP-022, acalabrutinib or zanubrutinib.

In some embodiments of the present application, drugs used for the chemotherapy comprise cyclophosphamide, ifosfamide, vincristine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, chlorambucil or azacitidine.

In some embodiments of the present application, the targeted drugs comprise bortezomib, rituximab, CHO-H01, ocaratuzumab, ibritumomab tiuxetan, ublituximab, obinutuzumab, brentuximab vedotin, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, BR-101801, tazemetostat or abexinostat.

In some embodiments of the present application, drugs used for the drug therapy comprise interferon, cyclophosphamide, ifosfamide, vincristine, vinorelbine, prednisone, prednisolone, doxorubicin, bortezomib, adriamycin, epirubicin, bleomycin, dexamethasone, methotrexate, cytarabine, carboplatin, cisplatin, bendamustine, fludarabine, mitoxantrone, etoposide, procarbazine, gemcitabine, carmustine, methylprednisolone, methylprednisolone sodium succinate, mesna, oxaliplatin, 5-fluorouracil, chlorambucil, dacarbazine, rituximab, CHO-H01, ocaratuzumab, ibritumomab tiuxetan, ublituximab, obinutuzumab, brentuximab vedotin, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, azacitidine, BR-101801, tazemetostat or abexinostat; preferably, drugs used for the drug therapy comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, rituximab, brentuximab vedotin, ibrutinib, ICP-022, acalabrutinib or zanubrutinib; more preferably, drugs used for the drug therapy comprise rituximab, ibrutinib, ICP-022, acalabrutinib or zanubrutinib.

In some embodiments of the present application, regimens for the chemotherapy comprise an ABVD regimen, an AVD regimen, a B regimen, a BA regimen, a BAC regimen, a BEACOPPesc regimen, a CDOP regimen, a CEOP regimen, a CEPP regimen, a CHOP regimen, a CHOEP regimen, a CIFOX regimen, a COP regimen, a COPE regimen, a CVP regimen, a DA-EPOCH regimen, a DHAP regimen, a DICE regimen, an EPOCH regimen, an ESHAP regimen, an FC regimen, an FM regimen, a GCVP regimen, a GDP regimen, a GDPE regimen, a GEMOX regimen, a GVD regimen, a HyperCVAD regimen, an ICE regimen, an IGEV regimen, an MA regimen, an MINE regimen, a miniBEAM regimen, an NCE regimen, a Stanford V regimen, a VCAP regimen, a high-dose cytarabine regimen, or the aforementioned regimen in combination with rituximab (which may be referred to as "R" when used for combination treatment); preferably, regimens for the chemotherapy comprise an ABVD regimen, an AVD regimen, a BEACOPPesc regimen, a BR regimen, a CDOP regimen, a CEOP regimen, a CEPP regimen, a CHOP regimen, a CHOEP regimen, a CIFOX regimen, a COP regimen, a CVP regimen, a DA-EPOCH-R regimen, a DHAP regimen, a DICE regimen, an EPOCH regimen, an ESHAP regimen, a FCR regimen, an FMR regimen, a GDP regimen, a GDPE regimen, a GEMOX regimen, a GVD regimen, an ICE regimen, an IGEV regimen, an MINE regimen, an NCE regimen, a miniBEAM regimen, R², an R-BAC regimen, an R-CDOP regimen, an R-CEOP regimen, an R-CEPP regimen, an R-CHOP regimen, an R-CHOEP regimen, an R-COPE regimen, an R-CVP regimen, an R-DHAP regimen, an R-DICE regimen, an R-EPOCH regimen, an R-ESHAP regimen, an R-GCVP regimen, an R-GDP regimen, an R-HyperCVAD regimen, an R-GDPE regimen, an R-GEMOX regimen, an R-ICE regimen, an R-MA regimen, an R-MINE regimen, an R-NCE regimen, a Stanford V regimen, an R-high-dose cytarabine regimen or a VR-CAP regimen.

In some embodiments of the present application, the radiotherapy is selected from the group consisting of total lymphoid irradiation (TLI) and sub-total lymphoid irradiation (STLI). In some embodiments of the present application, the radiotherapy comprises involved field radiation therapy (IFRT), involved nodal radiation therapy (INRT) or involved site radiation therapy (ISRT).

In some embodiments of the present application, the hematopoietic stem cell transplantation comprises autologous hematopoietic stem cell transplantation or allogeneic hematopoietic stem cell transplantation.

In some embodiments of the present application, the lymphoma is selected from the group consisting of Hodgkin's lymphoma and non-Hodgkin's lymphoma; optionally, the patient with the lymphoma has received treatment with one or more prior therapeutic regimens; optionally, the lymphoma is selected from relapsed or refractory lymphoma; optionally, the disease reoccurs after the patient with the lymphoma has received the treatment with the prior therapeutic regimen and achieved objective response, or the patient with the lymphoma has no objective response for the prior therapeutic regimen; optionally, the patient with the lymphoma is a patient with CD20-positive (CD20+), CD30-positive (CD30+), CD38-positive (CD38+) and/or ZAP70-positive (ZAP70+) lymphoma; optionally, the prior therapeutic regimens comprise drug therapy, radiotherapy or hematopoietic stem cell transplantation.

### Hodgkin's Lymphoma (HL)

In some embodiments of the present application, the lymphoma is selected from Hodgkin's lymphoma.

In some embodiments of the present application, the Hodgkin's lymphoma is selected from the group consisting of classical Hodgkin's lymphoma and nodular lymphocyte Hodgkin's lymphoma. In some embodiments of the present application, the classical Hodgkin's lymphoma is selected from the group consisting of nodular sclerosis classical Hodgkin's lymphoma, lymphocyte-rich classical Hodgkin's lymphoma, mixed cellularity classical Hodgkin's lymphoma and lymphocyte-depleted classical Hodgkin's lymphoma.

In some embodiments of the present application, the patient with the Hodgkin's lymphoma has received treatment with one or more prior therapeutic regimens. In some embodiments of the present application, the patient with the Hodgkin's lymphoma has received treatment with one, two, three, four or five prior therapeutic regimens.

In some embodiments of the present application, the patient with the Hodgkin's lymphoma is one who has received treatment with first-line, second-line or ≥ third-line prior therapeutic regimen.

In some embodiments of the present application, the disease reoccurs after the patient with the Hodgkin's lymphoma has received treatment with the prior therapeutic regimen and achieved objective response, or the patient with the Hodgkin's lymphoma has no objective response for the prior therapeutic regimen. In some embodiments of the present application, the no objective response refers to stable disease or disease progression during treatment.

In some embodiments of the present application, the patient with the Hodgkin's lymphoma is a patient with relapsed or refractory Hodgkin's lymphoma who has received treatment with a prior therapeutic regimen.

In some embodiments of the present application, the patient with the Hodgkin's lymphoma is a patient with CD20-positive (CD20+) or CD30-positive (CD30+) Hodgkin's lymphoma.

In some embodiments of the present application, the patient with the Hodgkin's lymphoma is a lymphoma patient who has received treatment with rituximab and/or a BTK inhibitor.

In some embodiments of the present application, the patient with the Hodgkin's lymphoma is a patient with relapsed or refractory Hodgkin's lymphoma who has received treatment with rituximab and/or a BTK inhibitor. In some embodiments of the present application, the patient with the Hodgkin's lymphoma is CD20-positive (CD20+) and is a patient with relapsed or refractory Hodgkin's lymphoma who has received treatment with rituximab.

In some embodiments of the present application, the prior therapeutic regimens for the Hodgkin's lymphoma comprise drug therapy, radiotherapy or hematopoietic stem cell transplantation.

In some embodiments of the present application, the drug therapy of the prior therapeutic regimens for the Hodgkin's lymphoma comprises interferon therapy, chemotherapy or targeted drug therapy.

In some embodiments of the present application, the targeted drug therapy of the prior therapeutic regimens for the Hodgkin's lymphoma comprises an anti-CD20 antibody, an anti-CD30 antibody or a BTK inhibitor.

In some embodiments of the present application, the anti-CD20 antibody of the prior therapeutic regimens for the Hodgkin's lymphoma comprises rituximab, CHO-H01, ocaratuzumab, ibritumomab tiuxetan, ublituximab or obinutuzumab.

In some embodiments of the present application, the anti-CD30 antibody of the prior therapeutic regimens for the Hodgkin's lymphoma comprises brentuximab vedotin.

In some embodiments of the present application, the BTK inhibitor of the prior therapeutic regimens for the Hodgkin's lymphoma comprises ibrutinib, ICP-022, acalabrutinib or zanubrutinib.

In some embodiments of the present application, drugs used for the chemotherapy of the prior therapeutic regimens for the Hodgkin's lymphoma comprise cyclophosphamide, ifosfamide, vincristine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone sodium succinate, mesna, oxaliplatin, 5-fluorouracil or azacitidine.

In some embodiments of the present application, targeted drugs of the prior therapeutic regimens for the Hodgkin's lymphoma comprise brentuximab vedotin, bortezomib, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, BR-101801, tazemetostat or abexinostat.

In some embodiments of the present application, drugs used for the drug therapy of the prior therapeutic regimens for the Hodgkin's lymphoma comprise interferon, cyclophosphamide, ifosfamide, vincristine, vinorelbine, prednisone, prednisolone, doxorubicin, bortezomib, adriamycin, epirubicin, bleomycin, dexamethasone, methotrexate, cytarabine, carboplatin, cisplatin, bendamustine, fludarabine, mitoxantrone, etoposide, procarbazine, gemcitabine, carmustine, methylprednisolone, methylprednisolone sodium succinate, mesna, oxaliplatin, 5-fluorouracil, dacarbazine, rituximab, CHO-H01, ocaratuzumab, ibritumomab tiuxetan, ublituximab, obinutuzumab, brentuximab vedotin, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, azacitidine, BR-101801, tazemetostat or abexinostat; preferably, drugs used for the drug therapy comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, rituximab, brentuximab vedotin, ibrutinib, ICP-022, acalabrutinib or zanubrutinib; more preferably, drugs used for the drug therapy comprise rituximab, ibrutinib, ICP-022, acalabrutinib or zanubrutinib. In some embodiments of the present application, chemotherapy regimens of the prior treatment for the Hodgkin's lymphoma comprise an ABVD regimen, an AVD regimen, a BEACOPPesc regimen, a CHOP regimen, a CVP regimen, a DHAP regimen, a DICE regimen, an EPOCH regimen, a GDP regimen, a GVD regimen, an ICE regimen, an IGEV regimen, an MINE regimen, a miniBEAM regimen or a Stanford V regimen.

In some embodiments of the present application, the hematopoietic stem cell transplantation comprises autologous hematopoietic stem cell transplantation or allogeneic hematopoietic stem cell transplantation.

In some embodiments of the present application, the radiotherapy is selected from the group consisting of total lymphoid irradiation (TLI) and sub-total lymphoid irradiation (STLI). In some embodiments of the present application, the radiotherapy comprises involved field radiation therapy (IFRT), involved nodal radiation therapy (INRT) or involved site radiation therapy (ISRT).

In some embodiments of the present application, the Hodgkin's lymphoma is selected from relapsed or refractory Hodgkin's lymphoma; optionally, the patient with the Hodgkin's lymphoma has received treatment with one or more prior therapeutic regimens; optionally, the disease reoccurs after the patient with the Hodgkin's lymphoma has received the treatment with the prior therapeutic regimen and achieved objective response, or the patient with the Hodgkin's lymphoma has no objective response for the prior therapeutic regimen; optionally, the patient with the Hodgkin's lymphoma is a patient with CD20-positive or CD30-positive Hodgkin's lymphoma. Optionally, the prior therapeutic regimens for the Hodgkin's lymphoma comprise drug therapy, radiotherapy or hematopoietic stem cell transplantation.

### Mantle Cell Lymphoma (MCL)

In some embodiments of the present application, the lymphoma or the non-Hodgkin's lymphoma is selected from mantle cell lymphoma.

In some embodiments of the present application, the mantle cell lymphoma is selected from the group consisting of classical mantle cell lymphoma, leukemic non-nodal mantle cell lymphoma and *in situ* mantle cell neoplasia.

In some embodiments of the present application, the mantle cell lymphoma is selected from relapsed or refractory mantle cell lymphoma.

In some embodiments of the present application, the patient with the mantle cell lymphoma has received treatment with one or more prior therapeutic regimens. In some embodiments of the present application, the patient with the mantle cell lymphoma has received treatment with one, two, three, four or five prior therapeutic regimens.

In some embodiments of the present application, the patient with the mantle cell lymphoma is one who has received treatment with first-line, second-line or ≥ third-line prior therapeutic regimen.

In some embodiments of the present application, the disease reoccurs after the patient with the mantle cell lymphoma has received treatment with the prior therapeutic regimen and achieved objective response, or the patient with the mantle cell lymphoma has no objective response for the prior therapeutic regimen. In some embodiments of the present application, the no objective response refers to stable disease or disease progression during treatment.

In some embodiments of the present application, the patient with the mantle cell lymphoma is one who has previously received systemic treatment ≥ first-line but ≤ fourth-line, but had no objective response (stable disease or disease progression during the treatment) after the therapeutic regimen accepted most recently or had disease progression after the treatment.

In some embodiments of the present application, the patient with the mantle cell lymphoma is a patient with relapsed or refractory mantle cell lymphoma who has received treatment with the prior therapeutic regimen.

In some embodiments of the present application, the patient with the mantle cell lymphoma is a patient with CD20-positive (CD20+) mantle cell lymphoma.

In some embodiments of the present application, the patient with the mantle cell lymphoma is one who has received treatment with rituximab and/or a BTK inhibitor.

In some embodiments of the present application, the patient with the mantle cell lymphoma is a patient with relapsed or refractory mantle cell lymphoma who has received treatment with rituximab and/or a BTK inhibitor. In some embodiments of the present application, the patient with the mantle cell lymphoma is CD20-positive (CD20+) and is a patient with relapsed or refractory mantle cell lymphoma who has received treatment with rituximab.

In some embodiments of the present application, the prior therapeutic regimens for the mantle cell lymphoma comprise drug therapy, radiotherapy or hematopoietic stem cell transplantation.

In some embodiments of the present application, the drug therapy of the prior therapeutic regimens for the mantle cell lymphoma comprises interferon therapy, chemotherapy or targeted drug therapy.

In some embodiments of the present application, the targeted drug therapy of the prior therapeutic regimens for the mantle cell lymphoma comprises an anti-CD20 antibody or a BTK inhibitor.

In some embodiments of the present application, the anti-CD20 antibody of the prior therapeutic regimens for the mantle cell lymphoma comprises rituximab, CHO-H01, ocaratuzumab, ibritumomab tiuxetan, ublituximab or obinutuzumab.

In some embodiments of the present application, the BTK inhibitor of the prior therapeutic regimens for the mantle cell lymphoma comprises ibrutinib, ICP-022, acalabrutinib or zanubrutinib.

In some embodiments of the present application, drugs used for the chemotherapy of the prior therapeutic regimens for the mantle cell lymphoma comprise cyclophosphamide, ifosfamide, vincristine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, or azacitidine.

In some embodiments of the present application, targeted drugs of the prior therapeutic regimens for the mantle cell lymphoma comprise bortezomib, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, BR-101801, tazemetostat or abexinostat.

In some embodiments of the present application, drugs used for the drug therapy of the prior therapeutic regimens for the mantle cell lymphoma comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, bendamustine, vincristine, bortezomib, carfilzomib, ixazomib, fludarabine, niraparib, umbralisib, lenalidomide, venetoclax or abexinostat; preferably, drugs used for the drug therapy comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, rituximab, ibrutinib, acalabrutinib, ICP-022 or zanubrutinib; more preferably, drugs used for the drug therapy comprise rituximab, ibrutinib, ICP-022, acalabrutinib or zanubrutinib. In some embodiments of the present application, chemotherapy regimens of the prior treatment for the mantle cell lymphoma comprise a B regimen, a CHOP regimen, a CIFOX regimen, a COP regimen, a DHAP regimen, an EPOCH regimen, an FC regimen, an FM regimen, a BAC regimen, a CHOP regimen, a HyperCVAD regimen, an ICE regimen, an MA regimen, a VCAP regimen, a high-dose cytarabine regimen, or the aforementioned regimen in combination with rituximab; preferably, the chemotherapy regimens comprise a BR regimen, a CHOP regimen, a COP regimen, a DHAP regimen, an FCR regimen, an FMR regimen, an R-BAC regimen, an R-CHOP regimen, an R-DHAP regimen, an R-HyperCVAD regimen, an R-ICE regimen, an R-MA regimen, an R-high-dose cytarabine regimen or a VR-CAP regimen.

In some embodiments of the present application, the hematopoietic stem cell transplantation comprises autologous hematopoietic stem cell transplantation or allogeneic hematopoietic stem cell transplantation.

In some embodiments of the present application, the radiotherapy is selected from the group consisting of total lymphoid irradiation (TLI) and sub-total lymphoid irradiation (STLI). In some embodiments of the present application, the radiotherapy comprises involved field radiation therapy (IFRT), involved nodal radiation therapy (INRT) or involved site radiation therapy (ISRT).

In some embodiments of the present application, a diagnostic report for a patient with the mantle cell lymphoma includes morphology and evidence of being cyclin D1 positive indicated by immunohistochemistry or that of t(11:14).

In some embodiments of the present application, a patient with the mantle cell lymphoma is t(11;14)-positive indicated by cytogenetic testing and/or cyclin D1-positive (highly expressed) indicated by immunohistochemistry. In some embodiments of the present application, the mantle cell lymphoma is selected from relapsed or refractory mantle cell lymphoma; optionally, the patient with the mantle cell lymphoma has received treatment with one or more prior therapeutic regimens; optionally, the disease reoccurs after the patient with the mantle cell lymphoma has received treatment with the prior therapeutic regimen and achieved objective response, or the patient with the mantle cell lymphoma has no objective response for the prior therapeutic regimen; optionally, the patient with the mantle cell lymphoma is a patient with CD20-positive mantle cell lymphoma; optionally, the prior therapeutic regimens for the mantle cell lymphoma comprise drug therapy, radiotherapy or hematopoietic stem cell transplantation; optionally, a diagnostic report for the patient with the mantle cell lymphoma includes morphology and evidence of being cyclin D1-positive indicated by immunohistochemistry or that of t(11:14).

In some embodiments of the present application, the mantle cell lymphoma is selected from relapsed or refractory mantle cell lymphoma; optionally, the patient with the mantle cell lymphoma has received treatment with one or more prior therapeutic regimens; optionally, the disease reoccurs after the patient with the mantle cell lymphoma has received treatment with the prior therapeutic regimen and achieved objective response, or the patient with the mantle cell lymphoma has no objective response for the prior therapeutic regimen; optionally, the patient with the mantle cell lymphoma is a patient with CD20-positive mantle cell lymphoma; optionally, the prior therapeutic regimens for the mantle cell lymphoma comprise drug therapy, radiotherapy or hematopoietic stem cell transplantation; optionally, the patient with the mantle cell lymphoma is t(11;14)-positive indicated by cytogenetic testing and/or cyclin D1-positive (highly expressed) indicated by immunohistochemistry.

### Follicular Lymphoma (FL)

In some embodiments of the present application, the lymphoma or the non-Hodgkin's lymphoma is selected from follicular lymphoma.

In some embodiments of the present application, the follicular lymphoma is selected from relapsed or refractory follicular lymphoma.

In some embodiments of the present application, the patient with the follicular lymphoma has received treatment with one or more prior therapeutic regimens. In some embodiments of the present application, the patient with the follicular lymphoma has received treatment with one, two, three, four or five prior therapeutic regimens.

In some embodiments of the present application, the patient with the follicular lymphoma is one who has received treatment with first-line, second-line or ≥ third-line prior therapeutic regimen.

In some embodiments of the present application, the patient with the follicular lymphoma is a patient with relapsed or refractory follicular lymphoma who has previously received ≥ second-line systemic treatment in which at least one of therapeutic regimens comprises rituximab.

In some embodiments of the present application, the disease reoccurs after the patient with the follicular lymphoma has received treatment with the prior therapeutic regimen and achieved objective response, or the patient with the follicular lymphoma has no objective response for the prior therapeutic regimen. In some embodiments of the present application, the no objective response refers to stable disease or disease progression during treatment.

In some embodiments of the present application, the patient with the follicular lymphoma is a patient with relapsed or refractory lymphoma who has received the prior therapeutic regimen.

In some embodiments of the present application, the patient with the follicular lymphoma is a patient with CD20-positive (CD20+) follicular lymphoma.

In some embodiments of the present application, the patient with the follicular lymphoma is one who has received treatment with rituximab and/or a BTK inhibitor.

In some embodiments of the present application, the patient with the follicular lymphoma is a patient with relapsed or refractory follicular lymphoma who has received treatment with rituximab and/or a BTK inhibitor.

In some embodiments of the present application, the patient with the follicular lymphoma is CD20-positive (CD20+) and is a patient with relapsed or refractory follicular lymphoma who has received treatment with rituximab.

In some embodiments of the present application, the prior therapeutic regimens for the follicular lymphoma comprise drug therapy, radiotherapy or hematopoietic stem cell transplantation.

In some embodiments of the present application, the drug therapy of the prior therapeutic regimens for the follicular lymphoma comprises interferon therapy, chemotherapy or targeted drug therapy.

In some embodiments of the present application, the targeted drug therapy of the prior therapeutic regimens for the follicular lymphoma comprises an anti-CD20 antibody or a BTK inhibitor.

In some embodiments of the present application, the anti-CD20 antibody of the prior therapeutic regimens for the follicular lymphoma comprises rituximab, CHO-H01, ocaratuzumab, ibritumomab tiuxetan, ublituximab or obinutuzumab.

In some embodiments of the present application, the BTK inhibitor of the prior therapeutic regimens for the follicular lymphoma comprises ibrutinib, ICP-022, acalabrutinib or zanubrutinib.

In some embodiments of the present application, drugs used for the chemotherapy of the prior therapeutic regimens for the follicular lymphoma comprise cyclophosphamide, ifosfamide, vincristine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil or azacitidine.

In some embodiments of the present application, targeted drugs of the prior therapeutic regimens for the follicular lymphoma comprise bortezomib, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, BR-101801, tazemetostat or abexinostat.

In some embodiments of the present application, drugs used for the drug therapy of the prior therapeutic regimens for the follicular lymphoma comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, vinorelbine, methylprednisolone, methylprednisolone sodium succinate, mesna, oxaliplatin, 5-fluorouracil, interferon, rituximab, CHO-H01, ocaratuzumab, ibritumomab tiuxetan, ublituximab, obinutuzumab, ibrutinib, acalabrutinib, ICP-022, zanubrutinib, vorinostat, azacitidine, BR-101801, umbralisib, tazemetostat or lenalidomide; preferably, drugs used for the drug therapy of the prior therapeutic regimens for the follicular lymphoma comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, interferon, rituximab, ibrutinib, acalabrutinib, ICP-022, zanubrutinib or lenalidomide; more preferably, drugs used for the drug therapy of the prior therapeutic regimens for the follicular lymphoma comprise rituximab, ibrutinib, ICP-022, acalabrutinib, zanubrutinib, bendamustine or lenalidomide.

In some embodiments of the present application, chemotherapy regimens of the prior therapeutic regimens for the follicular lymphoma comprise a B regimen, a CDOP regimen, a CHOP regimen, a CVP regimen, a DHAP regimen, a DICE regimen, an EPOCH regimen, an ICE regimen, a GEMOX regimen, an NCE regimen, or the aforementioned regimen in combination with rituximab; preferably, the chemotherapy regimens comprise a BR regimen, a CHOP regimen, a GEMOX regimen, an R-CDOP regimen, an R-CHOP regimen, an R-CVP regimen, an R-DHAP regimen, an R-DICE regimen, an R-EPOCH regimen, an R-GEMOX regimen, an R-ICE regimen or an R-NCE regimen.

In some embodiments of the present application, the hematopoietic stem cell transplantation comprises autologous hematopoietic stem cell transplantation or allogeneic hematopoietic stem cell transplantation.

In some embodiments of the present application, the radiotherapy is selected from the group consisting of total lymphoid irradiation (TLI) and sub-total lymphoid irradiation (STLI). In some embodiments of the present application, the radiotherapy comprises involved field radiation therapy (IFRT), involved nodal radiation therapy (INRT) or involved site radiation therapy (ISRT).

In some embodiments of the present application, the patient with the follicular lymphoma is a patient with grade 1-3a follicular lymphoma confirmed by histopathology.

In some embodiments of the present application, a diagnostic report for a patient with the follicular lymphoma includes t(14;18) translocation or overexpression of Bcl-2 protein.

In some embodiments of the present application, the follicular lymphoma is selected from relapsed or refractory follicular lymphoma; optionally, the patient with the follicular lymphoma has received treatment with one or more prior therapeutic regimens; optionally, the disease reoccurs after the patient with the follicular lymphoma has received treatment with the prior therapeutic regimen and achieved objective response, or the patient with the follicular lymphoma has no objective response for the prior therapeutic regimen; optionally, the patient with the follicular lymphoma is a patient with CD20-positive follicular lymphoma; optionally, the prior therapeutic regimens for the follicular lymphoma comprise drug therapy, radiotherapy or hematopoietic stem cell transplantation; optionally, the patient with the follicular lymphoma is a patient with grade 1-3a follicular lymphoma confirmed by histopathology; optionally, a diagnostic report for the patient with the follicular lymphoma includes t(14;18) translocation or overexpression of Bcl-2 protein.

### Diffuse Large B-Cell Lymphoma (DLBCL)

In some embodiments of the present application, the lymphoma or the non-Hodgkin's lymphoma is selected from diffuse large B-cell lymphoma.

In some embodiments of the present application, the diffuse large B-cell lymphoma is selected from relapsed or refractory diffuse large B-cell lymphoma.

In some embodiments of the present application, the patient with the diffuse large B-cell lymphoma has received treatment with one or more prior therapeutic regimens. In some embodiments of the present application, the patient with the diffuse large B-cell lymphoma has received treatment with one, two, three, four or five prior therapeutic regimens.

In some embodiments of the present application, the patient with the diffuse large B-cell lymphoma has received treatment with first-line, second-line or ≥ third-line prior therapeutic regimen.

In some embodiments of the present application, the patient with the diffuse large B-cell lymphoma has previously received treatment with at least second-line (comprising second-line, third-line, fourth-line or fifth-line) systemic therapeutic regimen.

In some embodiments of the present application, the patient with the diffuse large B-cell lymphoma has previously received at least second-line systemic therapeutic regimen, had disease progression during or after the most recent treatment, or had no objective response after adequate treatment, and at least one of the previous regimens comprises adequate treatment with rituximab or there is disease progression during treatment with rituximab.

In some embodiments of the present application, the disease reoccurs after the patient with the diffuse large B-cell lymphoma has received treatment with the prior therapeutic regimen and achieved objective response, or the patient with the diffuse large B-cell lymphoma has no objective response for the prior therapeutic regimen. In some embodiments of the present application, the no objective response refers to stable disease or disease progression during treatment.

In some embodiments of the present application, the patient with the diffuse large B-cell lymphoma is a patient with relapsed or refractory diffuse large B-cell lymphoma who has received prior therapeutic regimens.

In some embodiments of the present application, the patient with the diffuse large B-cell lymphoma is a patient with CD20-positive (CD20+) diffuse large B-cell lymphoma.

In some embodiments of the present application, the patient with the diffuse large B-cell lymphoma is one who has received treatment with rituximab and/or a BTK inhibitor.

In some embodiments of the present application, the patient with the diffuse large B-cell lymphoma is a patient with relapsed or refractory diffuse large B-cell lymphoma who has received treatment with rituximab and/or a BTK inhibitor.

In some embodiments of the present application, the patient with the diffuse large B-cell lymphoma is CD20-positive (CD20+) and is a patient with relapsed or refractory diffuse large B-cell lymphoma who has received treatment with rituximab.

In some embodiments of the present application, the prior therapeutic regimens for the diffuse large B-cell lymphoma comprise drug therapy, radiotherapy or hematopoietic stem cell transplantation.

In some embodiments of the present application, the drug therapy of the prior therapeutic regimens for the diffuse large B-cell lymphoma comprises interferon therapy, chemotherapy or targeted drug therapy.

In some embodiments of the present application, the targeted drug therapy of the prior therapeutic regimens for the diffuse large B-cell lymphoma comprises an anti-CD20 antibody or a BTK inhibitor.

In some embodiments of the present application, the anti-CD20 antibody of the prior therapeutic regimens for the diffuse large B-cell lymphoma comprises rituximab, CHO-H01, ocaratuzumab, ibritumomab tiuxetan, ublituximab or obinutuzumab.

In some embodiments of the present application, the BTK inhibitor of the prior therapeutic regimens for the diffuse large B-cell lymphoma comprises ibrutinib, ICP-022, acalabrutinib or zanubrutinib.

In some embodiments of the present application, drugs used for the chemotherapy of the prior therapeutic regimens for the diffuse large B-cell lymphoma comprise cyclophosphamide, ifosfamide, vincristine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil or azacitidine.

In some embodiments of the present application, targeted drugs of the prior therapeutic regimens for the diffuse large B-cell lymphoma comprise bortezomib, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, BR-101801, tazemetostat or abexinostat.

In some embodiments of the present application, drugs used for the drug therapy of the prior therapeutic regimens for the diffuse large B-cell lymphoma comprise cyclophosphamide, ifosfamide, vincristine, prednisone, prednisolone, doxorubicin, bortezomib, epirubicin, adriamycin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, interferon, rituximab, lenalidomide, CHO-H01, ocaratuzumab, ibritumomab tiuxetan, ublituximab, obinutuzumab, ibrutinib, acalabrutinib, ICP-022, zanubrutinib, vorinostat, azacitidine, BR-101801, umbralisib or tazemetostat; preferably, drugs used for the drug therapy of the prior therapeutic regimens for the diffuse large B-cell lymphoma comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, interferon, rituximab, lenalidomide, ibrutinib, acalabrutinib, ICP-022 or zanubrutinib; more preferably, drugs used for the drug therapy of the prior therapeutic regimens for the diffuse large B-cell lymphoma comprise rituximab, lenalidomide, ibrutinib, ICP-022, acalabrutinib or zanubrutinib.

In some embodiments of the present application, regimens used for the chemotherapy of the prior therapeutic regimens for the diffuse large B-cell lymphoma comprise a CDOP regimen, a GDP regimen, a GDPE regimen, a CEOP regimen, a CEPP regimen, a CHOP regimen, a CHOEP regimen, a DA-EPOCH regimen, a DHAP regimen, a DICE regimen, an ESHAP regimen, a GCVP regimen, a GDP regimen, a GEMOX regimen, a HyperCVAD regimen, an ICE regimen, an MINE regimen, or the aforementioned regimen in combination with rituximab; preferably, regimens used for the chemotherapy of the prior therapeutic regimens for the diffuse large B-cell lymphoma comprise a CEOP regimen, a CEPP regimen, a CHOP regimen, a DA-EPOCH-R regimen, a DHAP regimen, a DICE regimen, an ESHAP regimen, a GDP regimen, a GDPE regimen, a GEMOX regimen, an ICE regimen, an MINE regimen, an R-CDOP regimen, an R-CEOP regimen, an R-CEPP regimen, an R-CHOP regimen, an R-CHOEP regimen, an R-DHAP regimen, an R-ESHAP regimen, an R-GCVP regimen, an R-GDP regimen, an R-GDPE regimen, an R-GEMOX regimen, an R-HyperCVAD regimen, an R-ICE regimen or an R-MINE regimen.

In some embodiments of the present application, the hematopoietic stem cell transplantation comprises autologous hematopoietic stem cell transplantation or allogeneic hematopoietic stem cell transplantation.

In some embodiments of the present application, the radiotherapy is selected from the group consisting of total lymphoid irradiation (TLI) and sub-total lymphoid irradiation (STLI). In some embodiments of the present application, the radiotherapy comprises involved field radiation therapy (IFRT), involved nodal radiation therapy (INRT) or involved site radiation therapy (ISRT).

In some embodiments of the present application, the diffuse large B-cell lymphoma is selected from the group consisting of germinal center B-cell-like (GCB) diffuse large B-cell lymphoma, activated B-cell-like (ABC) diffuse large B-cell lymphoma and Type 3 diffuse large B-cell lymphoma.

In some embodiments of the present application, the diffuse large B-cell lymphoma is selected from relapsed or refractory diffuse large B-cell lymphoma; optionally, the patient with the diffuse large B-cell lymphoma has received treatment with one or more prior therapeutic regimens; optionally, the disease reoccurs after the patient with the diffuse large B-cell lymphoma has received treatment with the prior therapeutic regimen and achieved objective response, or the patient with the diffuse large B-cell lymphoma has no objective response for the prior therapeutic regimen; in some embodiments of the present application, the patient with the follicular lymphoma is a patient with CD20-positive follicular lymphoma; optionally, the prior therapeutic regimens for the diffuse large B-cell lymphoma include, but are not limited to, drug therapy, radiotherapy or hematopoietic stem cell transplantation.

### Small Lymphocytic Lymphoma (SLL) or Chronic Lymphocytic Leukemia (CLL)

In some embodiments of the present application, the lymphoma is selected from the group consisting of small lymphocytic lymphoma and chronic lymphocytic leukemia.

In some embodiments of the present application, the non-Hodgkin's lymphoma is selected from the group consisting of small lymphocytic lymphoma and chronic lymphocytic leukemia.

In some embodiments of the present application, the small lymphocytic lymphoma or the chronic lymphocytic leukemia is selected from relapsed or refractory small lymphocytic lymphoma or chronic lymphocytic leukemia.

In some embodiments of the present application, the patient with the small lymphocytic lymphoma or the chronic lymphocytic leukemia has received treatment with one or more prior therapeutic regimens. In some embodiments of the present application, the patient with the small lymphocytic lymphoma or the chronic lymphocytic leukemia has received treatment with one, two, three, four or five prior therapeutic regimens.

In some embodiments of the present application, the patient with the small lymphocytic lymphoma or the chronic lymphocytic leukemia has received treatment with first-line, second-line or ≥ third-line prior therapeutic regimen. In some embodiments of the present application, the patient with the small lymphocytic lymphoma or the chronic lymphocytic leukemia has previously received treatment with at least first-line (comprising first-line, second-line, third-line or fourth-line) systemic therapeutic regimen.

In some embodiments of the present application, the patient with the small lymphocytic lymphoma or the chronic lymphocytic leukemia has received treatment with at least first-line systemic therapeutic regimen.

In some embodiments of the present application, the patient with the small lymphocytic lymphoma or the chronic lymphocytic leukemia has previously received at least first-line systemic therapeutic regimen, had disease progression during or after the most recent treatment or had no objective response after adequate treatment.

In some embodiments of the present application, the patient with the small lymphocytic lymphoma or the chronic lymphocytic leukemia is a patient with relapsed or refractory small lymphocytic lymphoma or chronic lymphocytic leukemia who has received treatment with at least first-line systemic therapeutic regimen.

In some embodiments of the present application, the disease reoccurs after the patient with the small lymphocytic lymphoma or the chronic lymphocytic leukemia has received treatment with the prior therapeutic regimen and has objective response, or the patient with the small lymphocytic lymphoma or the chronic lymphocytic leukemia has no objective response for the prior therapeutic regimen. In some embodiments of the present application, the no objective response refers to stable disease or disease progression during treatment.

In some embodiments of the present application, the patient with the small lymphocytic lymphoma or the chronic lymphocytic leukemia is a patient with CD20-positive (CD20+), CD38-positive (CD38+) or ZAP70-positive (ZAP70+) small lymphocytic lymphoma or chronic lymphocytic leukemia.

In some embodiments of the present application, the patient with the small lymphocytic lymphoma or the chronic lymphocytic leukemia is CD20-positive (CD20+).

In some embodiments of the present application, the patient with the small lymphocytic lymphoma or the chronic lymphocytic leukemia is one who has received treatment with rituximab and/or a BTK inhibitor.

In some embodiments of the present application, the patient with the small lymphocytic lymphoma or the chronic lymphocytic leukemia is a patient with relapsed or refractory the small lymphocytic lymphoma or chronic lymphocytic leukemia who has received treatment with rituximab and/or a BTK inhibitor.

In some embodiments of the present application, the patient with the small lymphocytic lymphoma or the chronic lymphocytic leukemia is CD20-positive (CD20+) and is a patient with relapsed or refractory small lymphocytic lymphoma or chronic lymphocytic leukemia who has received treatment with rituximab.

In some embodiments of the present application, the patient with the small lymphocytic lymphoma or the chronic lymphocytic leukemia has del(11q), del(17p) and/or TP53 gene mutations.

In some embodiments of the present application, the prior therapeutic regimens for the small lymphocytic lymphoma or the chronic lymphocytic leukemia comprise drug therapy, radiotherapy or hematopoietic stem cell transplantation.

In some embodiments of the present application, the drug therapy of the prior therapeutic regimens for the small lymphocytic lymphoma or the chronic lymphocytic leukemia comprises interferon therapy, chemotherapy or targeted drug therapy.

In some embodiments of the present application, the targeted drug therapy of the prior therapeutic regimens for the small lymphocytic lymphoma or the chronic lymphocytic leukemia comprises an anti-CD20 antibody or a BTK inhibitor.

In some embodiments of the present application, the anti-CD20 antibody of the prior therapeutic regimens for the small lymphocytic lymphoma or the chronic lymphocytic leukemia comprises rituximab, CHO-H01, ocaratuzumab, ibritumomab tiuxetan, ublituximab or obinutuzumab.

In some embodiments of the present application, the BTK inhibitor of the prior therapeutic regimens for the small lymphocytic lymphoma or the chronic lymphocytic leukemia comprises ibrutinib, ICP-022, acalabrutinib or zanubrutinib.

In some embodiments of the present application, drugs used for the chemotherapy of the prior therapeutic regimens for the small lymphocytic lymphoma or the chronic lymphocytic leukemia comprise cyclophosphamide, ifosfamide, vincristine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, chlorambucil or azacitidine.

In some embodiments of the present application, targeted drugs of the prior therapeutic regimens for the small lymphocytic lymphoma or the chronic lymphocytic leukemia comprise bortezomib, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, BR-101801, tazemetostat or abexinostat.

In some embodiments of the present application, drugs used for the drug therapy of the prior therapeutic regimens for the small lymphocytic lymphoma or the chronic lymphocytic leukemia comprise interferon, cyclophosphamide, ifosfamide, vincristine, vinorelbine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, chlorambucil, rituximab, CHO-H01, ocaratuzumab, ibritumomab tiuxetan, ublituximab, obinutuzumab, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, azacitidine, BR-101801, tazemetostat or abexinostat; preferably, drugs used for the drug therapy comprise cyclophosphamide, ifosfamide, vincristine, prednisone, prednisolone, doxorubicin, bortezomib, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, rituximab, ibrutinib, ICP-022, acalabrutinib or zanubrutinib; more preferably, drugs used for the drug therapy comprise rituximab, ibrutinib, ICP-022, acalabrutinib or zanubrutinib.

In some embodiments of the present application, chemotherapy regimens for the prior treatment for the small lymphocytic lymphoma or the chronic lymphocytic leukemia comprise a B regimen, a BA regimen, a BAC regimen, a CDOP regimen, a CEOP regimen, a CEPP regimen, a CHOP regimen, a CIFOX regimen, a COP regimen, a COPE regimen, a CVP regimen, a DA-EPOCH regimen, a DHAP regimen, a DICE regimen, an EPOCH regimen, an ESHAP regimen, an FC regimen, an FM regimen, a GCVP regimen, a GDP regimen, a GDPE regimen, a GEMOX regimen, a HyperCVAD regimen, an ICE regimen, an MA regimen, an MINE regimen, an NCE regimen, a VCAP regimen, or the aforementioned regimen in combination with rituximab; preferably, regimens for the chemotherapy comprise a BR regimen, a CDOP regimen, a CEOP regimen, a CEPP regimen, a CHOP regimen, a CIFOX regimen, a COP regimen, a CVP regimen, a DA-EPOCH-R regimen, a DHAP regimen, a DICE regimen, an EPOCH regimen, an ESHAP regimen, an FCR regimen, an FMR regimen, a GDP regimen, a GDPE regimen, a GEMOX regimen, an ICE regimen, an MINE regimen, an NCE regimen, an R-BAC regimen, an R-CDOP regimen, an R-CEOP regimen, an R-CEPP regimen, an R-CHOP regimen, an R-CVP regimen, an R-DHAP regimen, an R-DICE regimen, an R-EPOCH regimen, an R-GCVP regimen, an R-HyperCVAD regimen, an R-ICE regimen, an R-MA regimen, an R-NCE regimen or a VR-CAP regimen.

In some embodiments of the present application, the radiotherapy is selected from the group consisting of total lymphoid irradiation (TLI) and sub-total lymphoid irradiation (STLI). In some embodiments of the present application, the radiotherapy comprises involved field radiation therapy (IFRT), involved nodal radiation therapy (INRT) or involved site radiation therapy (ISRT).

In some embodiments of the present application, the hematopoietic stem cell transplantation comprises autologous hematopoietic stem cell transplantation or allogeneic hematopoietic stem cell transplantation.

In some embodiments of the present application, the small lymphocytic lymphoma or the chronic lymphocytic leukemia is selected from relapsed or refractory small lymphocytic lymphoma or chronic lymphocytic leukemia; optionally, the patient with the small lymphocytic lymphoma or the chronic lymphocytic leukemia has received treatment with one or more prior therapeutic regimens; optionally, the disease reoccurs after the patient with the small lymphocytic lymphoma or the chronic lymphocytic leukemia has received treatment with the prior therapeutic regimen and achieved objective response, or the patient with the small lymphocytic lymphoma or the chronic lymphocytic leukemia has no objective response for the prior therapeutic regimen; optionally, the patient with the small lymphocytic lymphoma or the chronic lymphocytic leukemia is a patient with CD20-positive small lymphocytic lymphoma or chronic lymphocytic leukemia; optionally, the patient with the small lymphocytic lymphoma or the chronic lymphocytic leukemia has del(11q), del(17p) and/or TP53 gene mutations; optionally, the prior therapeutic regimens for the small lymphocytic lymphoma or the chronic lymphocytic leukemia comprise drug therapy, radiotherapy or hematopoietic stem cell transplantation.

### Administration regimen

In some embodiments of the present application, an administration cycle for treating lymphoma in a patient is 2-6 weeks. In some embodiments of the present application, the administration cycle for treating lymphoma in a patient is 2 weeks, 3 weeks, 4 weeks, 5 weeks, 6 weeks, or a range formed by any of the aforementioned values. In some embodiments of the present application, the administration cycle for treating lymphoma in a patient is 3 weeks or 4 weeks.

In some embodiments of the present application, a daily dose for treating lymphoma in a patient is selected from 1-100 mg. In some embodiments of the present application, the daily dose for treating lymphoma in a patient is selected from the group consisting of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, and ranges formed by any of the aforementioned values. In some embodiments of the present application, the daily dose for treating lymphoma in a patient is selected from the group consisting of 5-50 mg, 10-50 mg and 10-40 mg.

In some embodiments of the present application, the number of daily administrations for treating lymphoma in a patient is 1, 2 or 3.

In some embodiments of the present application, an administration frequency for treating lymphoma in a patient is once every two days.

In some embodiments of the present application, the administration regimen for treating lymphoma in a patient includes: an administration cycle of 2-6 weeks, a daily dose of 1-40 mg, and 1-3 administrations daily.

The compound of formula I, the stereoisomer thereof or the pharmaceutically acceptable salt thereof disclosed herein can be administered via multiple routes of administration including, but not limited to: oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular or intrathecal administrations. In one specific embodiment, the route is oral administration.

The route of administration can be determined according to factors such as the activity and toxicity of the drug, and tolerance of the patient. In some embodiments, the compound, the stereoisomer thereof or the pharmaceutically acceptable salt thereof disclosed herein is administered in an intermittent regimen.

The pharmaceutical composition disclosed herein can be prepared by combining the compound of formula I disclosed herein with a suitable pharmaceutically acceptable excipient, and can be formulated, for example, into a solid, semisolid, liquid or gaseous preparation.

In some embodiments of the present application, the pharmaceutical composition is a preparation suitable for oral administration, including tablets, capsules, powders, granules, dripping pills, pastes, pulvis, and the like, preferably tablets and capsules. The oral preparation may be prepared by a conventional method using a pharmaceutically acceptable carrier well known in the art. The pharmaceutically acceptable carrier includes diluents, binders, wetting agents, disintegrants, lubricants, and the like.

In some embodiments of the present application, the pharmaceutical composition is a single-dose pharmaceutical composition. In some embodiments, the pharmaceutical composition comprises 1 mg to 50 mg of the compound of formula I or the pharmaceutically acceptable salt thereof disclosed herein. In some embodiments, the pharmaceutical composition comprises 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg or 50 mg, or a range of any two of the foregoing values as endpoints or any value therein of the compound or the pharmaceutically acceptable salt thereof disclosed herein, for example, 2 mg to 50 mg, 10 mg to 40 mg, 5 mg to 30 mg, or 5 mg to 20 mg.

### TECHNICAL EFFECTS

The compound of formula I or the pharmaceutically acceptable salt thereof disclosed herein has favorable therapeutic efficacy in reducing the growth of lymphoma or even eliminating tumors, and provides good disease control rate to the treated patients to allow them to have longer survival (e.g., median survival, progression-free survival or overall survival), and longer duration of disease response. Meanwhile, the compound of formula I or the pharmaceutically acceptable salt thereof exhibits good safety in reducing lymphoma.

### Definitions and Description

Unless otherwise stated, the following terms used herein shall have the following meanings. A certain term, unless otherwise specifically defined, should not be considered uncertain or unclear, but construed according to its common meaning in the field. When referring to a trade name, it is intended to refer to its corresponding commercial product or its active ingredient.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases.

As used herein, the amount of the compound of formula I, e.g., the amount administered, the dose or the amount in the pharmaceutical composition, is calculated based on its free base form.

As used herein, if the compound in the pharmaceutical combination has, for example, at least one basic site, an acid addition salt may be formed. If needed, it may further form an acid addition salt with additional existing basic sites. A compound with at least one acidic group (for example, -COOH) can further form a salt with a base. A compound, for example, comprising both carboxyl and amino, can further form an inner salt.

The term "patient" is a mammal. In some embodiments, the patient is a human.

The term "pharmaceutical composition" refers to a mixture consisting of one or more of the compounds or the salts thereof, or the pharmaceutical combinations thereof disclosed herein and a pharmaceutically acceptable excipient. The pharmaceutical composition is intended to facilitate the administration of the compound or the pharmaceutical combination thereof disclosed herein to a patient.

The term "treat", "treating" or "treatment" usually refers to acquiring needed pharmacological effect and/or physiological effect. In terms of partially or fully stabilizing or curing the disease and/or a side effect of the disease, the effect can be therapeutic. As used herein, "treat", "treating" or "treatment" encompasses any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound disclosed herein for (i) treating a specific disease, condition or disorder; (ii) alleviating, relieving or eliminating one or more symptoms of a specific disease, condition or disorder, or (iii) delaying onset of one or more symptoms of a specific disease, condition or disorder described herein. The amount of the compound disclosed herein composing the "therapeutically effective amount" varies dependently on the compound, the condition and its severity, the administration regimen, and the age of the mammal to be treated, but can be determined routinely by those skilled in the art in accordance with their knowledge and the present disclosure.

The term "single dose" refers to the smallest unit of packaging containing a certain quantity of pharmaceutical product. For example, each tablet of drug is a single dose; in a box of seven capsules, each capsule is a single dose; or a vial of injection is a single dose.

In the context of cancer, the term "refractory" means that a particular cancer is resistant or non-responsive to therapy with a particular therapeutic agent. Cancers that are refractory to therapy with a particular therapeutic agent can begin when treatment with that particular therapeutic agent begins (i.e., does not respond as soon as exposure to the therapeutic agent begins), or develop resistance to the therapeutic agent during the first treatment period with the therapeutic agent or during subsequent treatments with the therapeutic agent. For example, being refractory to rituximab means that no response was achieved after adequate treatment with a rituximab-containing regimen (combination chemotherapy or single drug) or there is disease progression during the treatment or within 6 months of the end of the adequate treatment.

In the context of cancer, the term "relapsed" means that a disease reoccurs after objective response is achieved due to treatment with a certain therapeutic regimen. "Objective response" includes complete response and partial response. For example, being rituximab relapsed means that there is disease progression after response is achieved due to adequate treatment in which at least one regimen comprises rituximab.

In the context of cancer, the term "first-line therapy" refers to the first treatment of a disease. It is usually part of a standard set of treatments, such as post-operative chemotherapy and radiotherapy. The first-line therapy, when used alone, is recognized as the best therapy. If it does not cure the disease or causes serious side effects, other treatment methods may be added or used.

In the context of cancer, the term "second-line therapy" refers to a therapy given when the initial therapy (first-line therapy) is ineffective or ceases to function. The meaning of third-line therapy or multi-line therapy can be deduced accordingly.

As used herein, the phrase "has received treatment with a prior therapeutic regimen" or "has received a treatment method or drug therapy" means that the patient has previously received treatment with a corresponding treatment method, treatment method or drug. For example, "the patient with the lymphoma is a patient with relapsed or refractory lymphoma who has received treatment with rituximab" means that the patient with the lymphoma has previously been treated with rituximab.

As used herein, with respect to drugs used for the prior therapeutic regimens, reference may be made to the following or treatment guidelines or textbooks relating to medicine and pharmacy:
ABVD regimen: doxorubicin, bleomycin, vinblastine and dacarbazine.
AVD regimen: doxorubicin, vinblastine and dacarbazine.
B regimen: bendamustine.
BR regimen: bendamustine and rituximab.
BA regimen: bendamustine and azacitidine.
BEACOPPesc regimen: etoposide, doxorubicin, cyclophosphamide, vincristine, bleomycin, prednisone and procarbazine.
CDOP regimen: cyclophosphamide, doxorubicin, vincristine and prednisone.
CEOP regimen: cyclophosphamide, etoposide, vincristine and prednisone.
CEPP regimen: cyclophosphamide, etoposide, prednisolone and procarbazine.
CHOP regimen: cyclophosphamide, adriamycin/epirubicin, vincristine and prednisone. The CHOP regimen includes, but is not limited to, a CHOP-21 day regimen or a CHOP-14 day regimen.
CHOEP regimen: cyclophosphamide, adriamycin/epirubicin, vincristine, etoposide and prednisone (CHOP regimen in combination with etoposide).
CIFOX regimen: 5-fluorouracil and oxaliplatin.
COP regimen: cyclophosphamide, vincristine and prednisone.
COPE regimen: cyclophosphamide, vincristine, cisplatin and etoposide.
CVP regimen: cyclophosphamide, vincristine and prednisone.
DA-EPOCH regimen: etoposide, prednisone, vincristine, cyclophosphamide and adriamycin.
DA-EPOCH-R regimen: etoposide, prednisone, vincristine, cyclophosphamide, adriamycin and rituximab.
DHAP regimen: dexamethasone, high-dose cytarabine and cisplatin.
DICE regimen: dexamethasone, ifosfamide, cisplatin and etoposide.
EPOCH regimen: etoposide, prednisone, vincristine, cyclophosphamide and adriamycin.
ESHAP regimen: etoposide, methylprednisolone, high-dose cytarabine and cisplatin.
FCR regimen: fludarabine, cyclophosphamide and rituximab.
FC regimen: fludarabine and cyclophosphamide.
FM regimen: fludarabine and mitoxantrone.
FMR regimen: fludarabine, mitoxantrone and rituximab.
GCVP regimen: gemcitabine, cyclophosphamide, vincristine and prednisone.
GDP regimen: gemcitabine, dexamethasone and cisplatin.
GDPE regimen: gemcitabine, dexamethasone, cisplatin and etoposide.
GEMOX regimen: gemcitabine and oxaliplatin.
GVD regimen: gemcitabine, vinorelbine and doxorubicin.
HyperCVAD regimen: regimen A: cyclophosphamide, vincristine, adriamycin, dexamethasone and/or mesna; regimen B: methotrexate and cytarabine.
ICE regimen: ifosfamide, carboplatin and etoposide.
IGEV regimen: ifosfamide, gemcitabine and vinorelbine.
MA regimen: methotrexate and cytarabine.
MINE regimen: mesna, ifosfamide, mitoxantrone and etoposide.
miniBEAM regimen: carmustine, etoposide, cytarabine and melphalan.
NCE regimen: vinorelbine, cisplatin and etoposide.
R: rituximab.
R²: rituximab and lenalidomide.
R-: refers to the combination of rituximab with a therapeutic regimen. It includes, but is not limited to, the following:
   R-BAC regimen: rituximab, bendamustine and cytarabine.
   R-CDOP regimen: rituximab, cyclophosphamide, doxorubicin, vincristine and prednisone.
   R-CEOP regimen: rituximab, cyclophosphamide, etoposide, vincristine and prednisone.
   R-CEPP regimen: rituximab, cyclophosphamide, etoposide, prednisolone and procarbazine.
   R-CHOP regimen: rituximab, cyclophosphamide, adriamycin/epirubicin, vincristine and prednisone.
   R-CHOEP regimen: rituximab, cyclophosphamide, adriamycin/epirubicin, vincristine, etoposide and prednisone.
   R-COPE regimen: rituximab, cyclophosphamide, vincristine, cisplatin and etoposide.
   R-CVP regimen: rituximab, cyclophosphamide, vincristine and prednisone.
   R-DHAP regimen: dexamethasone, high-dose cytarabine and cisplatin.
   R-DICE regimen: rituximab, dexamethasone, ifosfamide, cisplatin and etoposide.
   R-EPOCH regimen: rituximab, etoposide, prednisone, vincristine, cyclophosphamide and adriamycin.
   R-ESHAP regimen: rituximab, etoposide, methylprednisolone, high-dose cytarabine and cisplatin.
   R-GCVP regimen: rituximab, gemcitabine, cyclophosphamide, vincristine and prednisolone.
   R-GDP regimen: rituximab, gemcitabine, dexamethasone and cisplatin.
   R-GDPE regimen: rituximab, gemcitabine, dexamethasone, cisplatin and etoposide.
   R-GEMOX regimen: rituximab, gemcitabine and oxaliplatin.
   R-HyperCVAD regimen: regimen A: rituximab, cyclophosphamide, vincristine, adriamycin, dexamethasone and/or mesna; regimen B: rituximab, methotrexate and cytarabine.
   R-ICE regimen: rituximab, ifosfamide, carboplatin and etoposide.
   R-MA regimen: rituximab, methotrexate and cytarabine.
   R-MINE regimen: rituximab, mesna, ifosfamide, mitoxantrone and etoposide.
   R-NCE regimen: rituximab, vinorelbine, cisplatin and etoposide.
   Stanford V regimen: doxorubicin, vinblastine, nitrogen mustard, vincristine, bleomycin, etoposide and prednisone. V-CAP regimen: bortezomib, cyclophosphamide, adriamycin and prednisone.
   VR-CAP regimen: bortezomib, rituximab, cyclophosphamide, adriamycin and prednisone.
   R-high-dose cytarabine: rituximab and high-dose cytarabine.

As used herein, the prednisolone may also be prednisone, and the two may be used interchangeably.

In the context of cancer, small lymphocytic lymphoma (SLL) and chronic lymphocytic leukemia (CLL) are different manifestations of the same disease, with SLL generally presenting no leukemic-like manifestations, and CLL being dominated by bone marrow and peripheral blood involvement. Thus, the protection scope of the present application for treating small lymphocytic lymphoma (SLL) also comprises chronic lymphocytic leukemia (CLL).

As used herein, the chemotherapy regimens belong to the prior art in this filed. Those skilled in the art would readily refer to treatment guidelines or related medical and pharmaceutical textbooks in the prior art (e.g., Chinese Society of Clinical Oncology (CSCO) diagnosis and treatment guidelines for malignant lymphoma 2019 or 2020) for details of a chemotherapy regimen (including but not limited to the drug used, the administration dose, or administration cycle). The above examples of drugs used for the chemotherapy regimens in the present application are exemplary, and the details of the chemotherapy regimens (including but not limited to the drug used, the administration dose, or administration cycle) will be subject to treatment guidelines or related medical and pharmaceutical textbooks.

As used herein, unless otherwise stated, the terms "comprise", "comprises" and "comprising" or equivalents thereof are open-ended statements and mean that elements, components and steps that are not specified may be included in addition to those listed.

All patents, patent applications and other identified publications are expressly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### DETAILED DESCRIPTION

The present invention will be illustrated in more detail through specific examples. The following examples are provided for illustrative purposes only, and are not intended to limit the present invention in any way.

### Example 1: Tablets of the Compound of Formula I

**Table 1. Formulation of tablets of the compound of formula I**

| Component | Amount | | | | | |
|---|---|---|---|---|---|---|
| | Specification 5 mg | | Specification 20 mg | | Specification 1 mg | |
| | mg | % | mg | % | mg | % |
| Compound of formula I | 5.0 | 5.0 | 20.0 | 5.0 | 1.0 | 1.0 |
| Microcrystalline cellulose | 25.0 | 25.0 | 100.0 | 25.0 | 25.0 | 25.0 |
| Mannitol | 63.0 | 63.0 | 252.0 | 63.0 | 67.0 | 67.0 |
| Croscarmellose sodium | 5 | 5 | 20 | 5 | 5 | 5 |
| Hydroxypropyl methylcellulose | 1.0 | 1.0 | 4.0 | 1.0 | 1.0 | 1.0 |
| Magnesium stearate | 1.0 | 1.0 | 4.0 | 1.0 | 1.0 | 1.0 |
| Weight of core tablet | 100 | 100 | 400 | 100 | 100 | 100 |

### Preparation method:

1) The compound of formula I, microcrystalline cellulose, mannitol and croscarmellose sodium were each fed into a grinding and sizing machine successively and then sieved, and materials were then collected and premixed to obtain a premixed material.
2) Hydroxypropyl methylcellulose was formulated into an aqueous solution to be used as a binder.
3) The premixed material in the step 1) was transferred into a wet granulation pot, the binder obtained in the step 2) was added, and the granulation was started.
4) The soft and wet materials obtained were subjected to sizing and drying, and then magnesium stearate was added to be mixed together.
5) Tableting was performed.

Optionally, the resulting tablets were coated.

The compound of formula I was prepared according to the method disclosed in WO2015192760.

### Example 2: Lymphoma

### 2.1 Administration regimen

Method of administration: orally administered once daily (QD administration), with 28 consecutive days of administration as one treatment cycle.

Drug: tablet of the compound of formula **I,** 1 mg or 5 mg.

### 2.2 Enrollment criteria

1) Unambiguously diagnosed as relapsed or refractory lymphoma by pathology and/or cytology;
2) Has at least one measurable target lesion other than brain lesions confirmed by imaging in screening phase (evaluated according to the evaluation criteria revised by the Lugano conference, 2014 edition);
3) Aged 18-75 years;
4) ECOG (PS) score ≤ 1;
5) Expected survival time ≥ 3 months;
6) Main organ functions in the screening phase meet the following criteria:
   Criteria for blood routine examination (no growth factor used or no blood transfusion conducted within 7 days):
      Absolute value of neutrophil count (ANC) ≥ 1.5×10⁹/L;
      Platelet (PLT) ≥ 75×10⁹/L; hemoglobin (Hb) ≥ 90 g/L;
   Criteria for blood biochemical examination:
      Alanine transaminase (ALT) and aspartate transferase (AST) ≤ 1.5 × ULN (for patients with tumor liver metastasis, ≤ 3 × ULN);
      Glycosylated hemoglobin (HbA1c) ≤ 8.5%;
      Lipase ≤ 1.5 × ULN;
      Serum total bilirubin ≤ 1.5 × ULN;
      Serum creatinine (Cr) ≤ 1.5 × ULN;
7) Doppler ultrasound evaluation: left ventricular ejection fraction (LVEF) ≥ lower limit of normal (50%);
8) Female subjects should agree to take contraceptive measures (such as intrauterine devices [IUD], contraceptives or condoms) during the study and for 6 months after the study; serum or urine pregnancy test results should be negative within 7 days before enrollment, and the subjects must not be breastfeeding; male subjects should agree to take contraceptive measures during the study and for 6 months after the study; and
9) Voluntary participation, written informed consent and good compliance.

### 2.3 Evaluation method and index

The efficacy was evaluated according to the revised evaluation criteria of the Lugano conference, 2014 edition. Primary evaluation indexes of efficacy: objective response rate (ORR), i.e., (CR + PR cases)/total cases, including cases of complete response (CR) and partial response (PR).

Secondary evaluation indexes of efficacy: progression-free survival (PFS), overall survival (OS), disease control rate (DCR) and duration of response (DOR).

### 2.4 Results of trial

When 22 patients with lymphoma were enrolled, 18 patients could be evaluated for efficacy, wherein 2 patients achieved CR and 9 patients achieved PR (all being the best efficacy). ORR was 73% (11/15) in patients in the 10 mg/qd and higher-dose group, and was 100% (4/4) in patients in the trial 20 mg/qd group. Notably, the ORR for follicular lymphoma (FL) was 75% (6/8) in the 10 mg/qd and higher-dose group, and all the two follicular lymphoma patients in the 20 mg/qd group achieved PR. All the two mantle cell lymphoma patients also achieved PR and they were relapsed or refractory patients after BTK inhibitor treatment.

**Table 2. History of lymphoma patients and treatment history**

| Patient No. | Disease | Treatment history |
|---|---|---|
| R1018 | FL | 1. R-CHOP; 2. R-NCE; 3. CHOP; 4. R-DICE |
| R2008 | FL | 1. R-CHOP; 2. R-EPOCH; 3. R-ICE; 4. GEMOX; 5. R² |
| R1006 | FL | 1. CHOP twice |
| R2011 | FL | 1. R-CHOP; 2. R-GEMOX |
| R1009 | DLBCL | 1. CDOP; 2. R-DICE; 3. autologous hematopoietic stem cell transplantation |
| R2006 | DLBCL | 1. CHOP; 2. R-GDPE |
| R2009 | MCL | 1. CHOP; 2. R-CHOP; 3. R maintenance |

| | | |
|---|---|---|
| Note: R² is rituximab + lenalidomide in combination; R maintenance is the administration of rituximab to the patient once every four weeks. | | |

**Table 3. Evaluation results of efficacy in lymphoma patients**

| Patient No. | Disease | Method of administration | Administration cycle | Best efficacy | Change in lesion |
|---|---|---|---|---|---|
| R1018 | FL | 20 mg/bid | C2D28 | Small SD | -33.0% |
| R2008 | FL | 10 mg/bid | C2D3 | Small SD | -42.0% |
| R1006 | FL | 10 mg/qd | C5D28 | PR | -84.0% |
| R2011 | FL | 20 mg/bid | C1D14 | PR | -94.0% |
| R1009 | DLBCL | 10 mg/qd | C7D28 | Small SD | -37.5% |
| R2006 | DLBCL | 10 mg/qd | C3D28 | CR | -100.0% |
| R2009 | MCL | 10 mg/bid | C3D28 | PR | -71.0% |

| | | | | | |
|---|---|---|---|---|---|
| Note: "Small SD" means that at the time of efficacy evaluation, a lesion shrinks in size compared with a baseline level, but does not meet the criteria for PR. | | | | | |

When 35 lymphoma patients were enrolled, 31 lymphoma patients were evaluated for efficacy, and ORR was 71% (22/31). In terms of safety, the major grade 3 adverse event and dose-limiting toxicity (DLT) associated with study drugs were hyperglycemia, which could be controlled and returned to baseline level by withdrawal of drug or symptom-oriented treatment. 20 patients had been enrolled in the 20 mg QD dose group and exhibited good overall safety and tolerance, and there was only 1 case of adverse event, a grade 3 hyperglycemia.

When 50 lymphoma patients were enrolled, 43 lymphoma patients were evaluated for efficacy, and ORR was 65% (28/43). See table 4 for specific results:

**Table 4. Evaluation results of efficacy in lymphoma patients**

| Tumor type | Number of patients | ORR, n (%) | CR, n (%) |
|---|---|---|---|
| Lymphoma | 43 | 28 (65%) | 4 (9.3%) |
| NHL | 41 | 28 (68%) | 4 (9.8%) |
| CLL | 2 | 2 (100%) | ---- |
| FL | 20 | 15 (75%) | 2 (10.0%) |
| MCL | 5 | 5 (100%) | ---- |
| DLBCL | 8 | 4 (50.0%) | 2 (25.0%) |

The results of the trial described above show that the compound of formula I has good therapeutic effect on lymphoma and has good safety.

### Example 3: Mantle Cell Lymphoma

### 3.1 Administration regimen

Method of administration: orally administered once daily, 20 mg each time, with 28 consecutive days of administration as one cycle.

Drug: tablet of the compound of formula I, 5 mg or 20 mg.

### 3.2 Enrollment criteria

1) Histopathologically confirmed as relapsed/refractory mantle cell lymphoma, with the diagnostic report required to include evidence of being t(11;14)-positive indicated by cytogenetic testing and/or high cyclin D1 expression indicated by immunohistochemistry;
2) Has previously received systemic treatment ≥ first-line but ≤ fourth-line, had no objective response (stable disease or disease progression during the treatment) achieved after the therapeutic regimen accepted most recently or had disease progression after the treatment;
3) At least one measurable target lesion present (evaluated according to the Lugano evaluation criteria, 2014 edition);
4) Aged 18-75 years; ECOG (PS) score: 0-2; expected survival time ≥ 3 months;
5) Main organ functions in the screening phase meet the following criteria:
   Criteria for blood routine examination (no growth factor used or no blood transfusion conducted within 7 days):
      Absolute value of neutrophil count (ANC) ≥ 1.0×10⁹/L;
      Platelet (PLT) ≥ 75×10⁹/L (for patients with lymphoma bone marrow infiltration, the criterion lowered to be 50×10⁹/L);
      Hemoglobin (Hb) ≥ 80 g/L;
   Criteria for blood biochemical examination:
      Alanine transaminase (ALT) and aspartate transferase (AST) ≤ 2.5 × ULN (for patients with tumor liver metastasis, ≤ 5 × ULN);
      Glycosylated hemoglobin (HbA1c) ≤ 8.5%;
      Serum amylase and lipase ≤ 1.5 × ULN;
      Total bilirubin (TBIL) in serum ≤ 1.5 × ULN (for patients with Gilbert syndrome, ≤ 3 × ULN);
      Blood coagulation: activated partial thromboplastin time (APTT), international normalized ratio (INR),
      prothrombin time (PT) ≤ 1.5 × ULN;
      Serum creatinine (Cr) ≤ 1.5 × ULN or creatinine clearance ≥ 50 mL/min;
6) Doppler ultrasound evaluation: left ventricular ejection fraction (LVEF) ≥ 50%;
7) Female subjects should agree to take contraceptive measures (such as intrauterine devices [IUD], contraceptives or condoms) during the study and for 6 months after the study; serum or urine pregnancy test results should be negative within 7 days before enrollment, and the subjects must not be breastfeeding; male subjects should agree to take contraceptive measures during the study and for 6 months after the study; and
8) Voluntary participation, written informed consent and good compliance.

### 3.3 Evaluation method and index

The efficacy was evaluated according to the revised evaluation criteria of the Lugano conference, 2014 edition. Primary evaluation indexes of efficacy: objective response rate (ORR), i.e., (CR + PR cases)/total cases, including cases of complete response (CR) and partial response (PR).

Secondary evaluation indexes of efficacy: progression-free survival (PFS), overall survival (OS), disease control rate (DCR) and duration of response (DOR).

### 3.4 Results of trial

Efficacy was evaluated in 21 patients with mantle cell lymphoma (7 patients had been previously treated with a BTK inhibitor, or 14 patients had been previously treated with rituximab, where there was the case that some patients had been previously treated with both a BTK inhibitor and rituximab), ORR was 76% (16 cases), and CR was 9.5% (2 cases). Five of the 7 patients previously treated with a BTK inhibitor reached ORR. The results show that the compound of formula I has a good therapeutic effect on mantle cell lymphoma. Meanwhile, the compound of formula I has good safety in treatment.

### Example 4: Follicular Lymphoma

### 4.1 Administration regimen

Method of administration: orally administered once daily, 20 mg each time, with 28 consecutive days of administration as one cycle.

Drug: tablet of the compound of formula **I,** 5 mg or 20 mg.

### 4.2 Enrollment criteria

1) Histopathologically confirmed as a patient with grade 1-3a follicular lymphoma (FL);
2) Must be a patient with relapsed or refractory FL who has previously received ≥ second-line systemic treatment (at least 1 regimen comprising rituximab);
3) At least one measurable target lesion present (evaluated according to the Lugano evaluation criteria, 2014 edition);
4) No gender limitation, aged ≥ 18 years; ECOG (PS) score: 0-2; expected survival time ≥ 3 months;
5) Main organ functions in the screening phase meet the following criteria:
   Criteria for blood routine examination (no growth factor used or no blood transfusion conducted within 7 days):
      Absolute value of neutrophil count (ANC) ≥ 1.0×10⁹/L;
      Platelet (PLT) ≥ 75×10⁹/L (for patients with lymphoma bone marrow infiltration, the criterion lowered to be 50×10⁹/L);
      Hemoglobin (Hb) ≥ 80 g/L;
   Criteria for blood biochemical examination:
      Alanine transaminase (ALT) and aspartate transferase (AST) ≤ 2.5 × ULN (for patients with liver involvement of lymphoma or biliary obstruction, ≤ 5 × ULN);
      Serum total bilirubin (TBIL) ≤ 1.5 × ULN;
      Blood coagulation: activated partial thromboplastin time (APTT), international normalized ratio (INR),
      prothrombin time (PT) ≤ 1.5 × ULN;
      Serum creatinine (Cr) ≤ 1.5 × ULN or creatinine clearance ≥ 50 mL/min;
6) Doppler ultrasound evaluation: left ventricular ejection fraction (LVEF) ≥ 50%;
7) Female subjects should agree to take contraceptive measures (such as intrauterine devices [IUD], contraceptives or condoms) during the study and for 6 months after the study; serum or urine pregnancy test results should be negative within 7 days before enrollment, and the subjects must not be breastfeeding; male subjects should agree to take contraceptive measures during the study and for 6 months after the study; and
8) Voluntary participation, written informed consent and good compliance.

### 4.3 Evaluation method and index

The efficacy was evaluated according to the revised evaluation criteria of the Lugano conference, 2014 edition. Primary evaluation indexes of efficacy: objective response rate (ORR), i.e., (CR + PR cases)/total cases, including cases of complete response (CR) and partial response (PR).

Secondary evaluation indexes of efficacy: progression-free survival (PFS), overall survival (OS), disease control rate (DCR) and duration of response (DOR).

### 4.4 Results of trial

Efficacy was evaluated in 20 patients with follicular lymphoma, and ORR was 70% (14 cases) and CR was 10.0% (2 cases). The results show that the compound of formula I has good therapeutic effect on follicular lymphoma. Meanwhile, the compound of formula I has good safety in treatment.

### Example 5: Diffuse Large B-Cell Lymphoma

### 5.1 Administration regimen

Method of administration: orally administered once daily, 20 mg each time, with 21 consecutive days of administration as one cycle.

Drug: tablet of the compound of formula I, 5 mg or 20 mg.

### 5.2 Enrollment criteria

1) Histopathologically confirmed as relapsed/refractory diffuse large B-cell lymphoma (DLBCL);
2) Has previously received at least second-line systemic therapeutic regimen, had disease progression during or after the most recent treatment, or had no objective response confirmed after adequate treatment, with at least one of the previous regimens comprising adequate treatment with rituximab or disease progression present during treatment with rituximab;
3) At least one measurable target lesion present (evaluated according to the Lugano evaluation criteria, 2014 edition);
4) Main organ functions in the screening phase meet the following criteria:
   Criteria for blood routine examination (no growth factor used or no blood transfusion conducted within 7 days):
      Absolute value of neutrophil count (ANC) ≥ 1.0×10⁹/L;
      Platelet (PLT) ≥ 75×10⁹/L (for patients with lymphoma bone marrow infiltration, 50× 10⁹/L acceptable);
      Hemoglobin (Hb) ≥ 80 g/L;
   Criteria for blood biochemical examination:
      Alanine transaminase (ALT) and aspartate transferase (AST) ≤ 2.5 × ULN (for patients with liver involvement of lymphoma or biliary obstruction, ≤ 5 × ULN);
      Serum total bilirubin (TBIL) ≤ 1.5 × ULN;
      Blood coagulation: activated partial thromboplastin time (APTT), international normalized ratio (INR),
      prothrombin time (PT) ≤ 1.5 × ULN;
      Serum creatinine (Cr) ≤ 1.5 × ULN or creatinine clearance ≥ 50 mL/min;
5) Female subjects should agree to take contraceptive measures (such as intrauterine devices [IUD], contraceptives or condoms) during the study and for 6 months after the study; serum or urine pregnancy test results should be negative within 7 days before enrollment, and the subjects must not be breastfeeding; male subjects should agree to take contraceptive measures during the study and for 6 months after the study; and
6) Voluntary participation, written informed consent and good compliance.

### 5.3 Evaluation method and index

The efficacy was evaluated according to the revised evaluation criteria of the Lugano conference, 2014 edition. Primary evaluation indexes of efficacy: objective response rate (ORR), i.e., (CR + PR cases)/total cases, including cases of complete response (CR) and partial response (PR).

Secondary evaluation indexes of efficacy: progression-free survival (PFS), overall survival (OS), disease control rate (DCR) and duration of response (DOR).

### 5.4 Results of trial

The compound of formula **I** has good therapeutic effect on diffuse large B-cell lymphoma. Meanwhile, the compound of formula **I** has good safety in treatment.

### Example 6: Small Lymphocytic Lymphoma or Chronic Lymphocytic Leukemia

### 6.1 Administration regimen

Method of administration: orally administered once daily, 20 mg each time, with 28 consecutive days of administration as one cycle.

Drug: tablet of the compound of formula **I,** 5 mg or 20 mg.

### 6.2 Enrollment criteria

1) Voluntary participation and written informed consent;
2) No gender limitation, aged ≥ 18 years; ECOG (PS) score: 0-2; expected survival time ≥ 3 months;
3) Chronic lymphocytic leukemia/small lymphocytic lymphoma confirmed by flow cytometry or pathology and meeting at least 1 of the criteria for active disease requiring treatment in IWCLL2008;
4) Has previously received at least first-line systemic therapeutic regimen, had disease progression during or after the most recent treatment, or had no objective response after adequate treatment;
5) At least one measurable tumor lesion (the length of a lesion in the node > 15 mm, and the length of a lesion outside the node > 10 mm) present in 2 vertical directions evaluated by CT or MRI;
6) Main organ functions in the screening phase meet the following criteria:
   Criteria for blood routine examination (no growth factor used or no blood transfusion conducted within 7 days):
      Absolute value of neutrophil count (ANC) ≥ 1.0×10⁹/L;
      Platelet (PLT) ≥ 75×10⁹/L (for patients with lymphoma bone marrow infiltration, ≥50×10⁹/L acceptable); hemoglobin (Hb) ≥ 80 g/L;
   Criteria for blood biochemical examination:
      Alanine transaminase (ALT) and aspartate transferase (AST) ≤ 2.5 × ULN (for patients with liver involvement of lymphoma or biliary obstruction, ≤ 5 × ULN);
      Serum total bilirubin (TBIL) ≤ 1.5 × ULN;
      Serum creatinine (Cr) ≤ 1.5 × ULN or creatinine clearance ≥ 50 mL/min;
   Blood coagulation:
      activated partial thromboplastin time (APTT), international normalized ratio (INR), prothrombin time (PT) ≤ 1.5 × ULN;
7) Female subjects should agree to take contraceptive measures (such as intrauterine devices [IUD], contraceptives or condoms) during the study and for 6 months after the study; serum or urine pregnancy test results should be negative within 7 days before enrollment, and the subjects must not be breastfeeding; male subjects should agree to take contraceptive measures during the study and for 6 months after the study.

### 6.3 Evaluation method and index

The efficacy was evaluated with reference to IWCLL2008 evaluation criteria and the evaluation criteria revised by the Lugano conference, 2014 edition.

Primary evaluation indexes of efficacy: objective response rate (ORR), i.e., (CR + PR cases)/total cases, including cases of complete response (CR) and partial response (PR).

Secondary evaluation indexes of efficacy: progression-free survival (PFS), overall survival (OS), disease control rate (DCR) and duration of response (DOR).

### 6.4 Results of trial

The compound of formula **I** has good treatment effect on small lymphocytic lymphoma or chronic lymphocytic leukemia. Meanwhile, the compound of formula **I** has good safety in treatment.

## Claims

1. A compound of formula **I** or a pharmaceutically acceptable salt thereof for use in treating lymphoma in a patient:

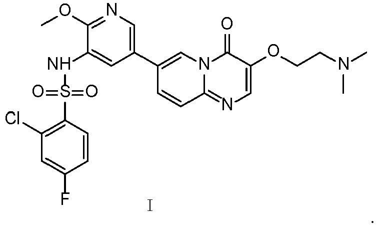

2. The compound of formula **I** or the pharmaceutically acceptable salt thereof according to claim 1, wherein the lymphoma is selected from the group consisting of Hodgkin's lymphoma and non-Hodgkin's lymphoma.

3. The compound of formula **I** or the pharmaceutically acceptable salt thereof according to claim 1 or 2, wherein the lymphoma is selected from the group consisting of B-cell lymphoma and T-cell lymphoma; or, the lymphoma is selected from the group consisting of classical Hodgkin's lymphoma (CHL), nodular lymphocyte Hodgkin's lymphoma, mantle cell lymphoma (MCL), follicular lymphoma (FL), diffuse large B-cell lymphoma (DLBCL), small lymphocytic lymphoma (SLL) and chronic lymphocytic leukemia (CLL).

4. The compound of formula **I** or the pharmaceutically acceptable salt thereof according to claim 2, wherein the non-Hodgkin's lymphoma is selected from the group consisting of B-cell lymphoma and T-cell lymphoma; or, the non-Hodgkin's lymphoma is selected from the group consisting of mantle cell lymphoma (MCL), follicular lymphoma (FL), diffuse large B-cell lymphoma (DLBCL), small lymphocytic lymphoma (SLL) and chronic lymphocytic leukemia (CLL).

5. The compound of formula **I** or the pharmaceutically acceptable salt thereof according to claim 2, wherein the Hodgkin's lymphoma is selected from the group consisting of classical Hodgkin's lymphoma and nodular lymphocyte Hodgkin's lymphoma; preferably, the classical Hodgkin's lymphoma is selected from the group consisting of nodular sclerosis classical Hodgkin's lymphoma, lymphocyte-rich classical Hodgkin's lymphoma, mixed cellularity classical Hodgkin's lymphoma and lymphocyte-depleted classical Hodgkin's lymphoma.

6. The compound of formula **I** or the pharmaceutically acceptable salt thereof according to any one of claims 1-5, wherein the patient with the lymphoma has received treatment with one or more prior therapeutic regimens; optionally, the patient with the lymphoma has received treatment with one, two, three, four or five prior therapeutic regimens.

7. The compound of formula **I** or the pharmaceutically acceptable salt thereof according to any one of claims 1-6, wherein the lymphoma is selected from relapsed or refractory lymphoma; optionally, the disease reoccurs after the patient with the lymphoma has received the treatment with the prior therapeutic regimen and achieved objective response, or the patient with the lymphoma has no objective response for the prior therapeutic regimen.

8. The compound of formula **I** or the pharmaceutically acceptable salt thereof according to any one of claims 1-7, wherein the patient with the lymphoma is one who has received treatment with rituximab and/or a BTK inhibitor; optionally, the patient with the lymphoma is a patient with relapsed or refractory lymphoma who has received treatment with rituximab and/or a BTK inhibitor.

9. The compound of formula **I** or the pharmaceutically acceptable salt thereof according to any one of claims 1-8, wherein the patient with lymphoma is a patient with CD20-positive, CD30-positive, CD38-positive and/or ZAP70-positive lymphoma; optionally, the patient with the lymphoma is CD20-positive and is a patient with relapsed or refractory lymphoma who has received treatment with rituximab.

10. The compound of formula **I** or the pharmaceutically acceptable salt thereof according to claim 6, wherein the prior therapeutic regimens comprise drug therapy, radiotherapy or hematopoietic stem cell transplantation.

11. The compound of formula I or the pharmaceutically acceptable salt thereof according to claim 10, wherein the drug therapy comprises interferon therapy, chemotherapy or targeted drug therapy; preferably, the radiotherapy is selected from the group consisting of total lymphoid irradiation and sub-total lymphoid irradiation; optionally, the radiotherapy comprises involved field radiation therapy, involved nodal radiation therapy or involved site radiation therapy; the hematopoietic stem cell transplantation comprises autologous hematopoietic stem cell transplantation or allogeneic hematopoietic stem cell transplantation.

12. The compound of formula **I** or the pharmaceutically acceptable salt thereof according to claim 10 or 11, wherein a drug used for the drug therapy is selected from one or more of the group consisting of: interferon, cyclophosphamide, ifosfamide, vincristine, vinorelbine, prednisone, prednisolone, doxorubicin, bortezomib, adriamycin, epirubicin, bleomycin, dexamethasone, methotrexate, cytarabine, carboplatin, cisplatin, bendamustine, fludarabine, mitoxantrone, etoposide, procarbazine, gemcitabine, carmustine, methylprednisolone, methylprednisolone sodium succinate, mesna, oxaliplatin, 5-fluorouracil, chlorambucil, dacarbazine, rituximab, CHO-H01, ocaratuzumab, ibritumomab tiuxetan, ublituximab, obinutuzumab, brentuximab vedotin, ibrutinib, ICP-022, acalabrutinib, zanubrutinib, palbociclib, abemaciclib, temsirolimus, everolimus, carfilzomib, ixazomib, niraparib, umbralisib, lenalidomide, venetoclax, vorinostat, azacitidine, BR-101801, tazemetostat or abexinostat, or combinations thereof.

13. The compound of formula **I** or the pharmaceutically acceptable salt thereof according to any one of claims 1-12, wherein an administration cycle for treating the lymphoma in the patient is 2-6 weeks.

14. The compound of formula **I** or the pharmaceutically acceptable salt thereof according to any one of claims 1-13, wherein a daily dose for treating the lymphoma in the patient is selected from 1-100 mg.

15. The compound of formula **I** or the pharmaceutically acceptable salt thereof according to any one of claims 1-14, wherein the number of daily administrations for treating the lymphoma in the patient is 1, 2 or 3.

16. Use of a compound of formula **I** or a pharmaceutically acceptable salt thereof for preparing a medicament or a pharmaceutical composition for treating lymphoma in a patient.

17. Use of a compound of formula **I** or a pharmaceutically acceptable salt thereof in treating lymphoma in a patient.

18. A method for treating lymphoma in a patient, comprising administering to the patient a compound of formula **I** or a pharmaceutically acceptable salt thereof.
